(19) **Europäisches Patentamt**
**European Patent Office**
**Office européen des brevets**

(11) **EP 3 471 752 B1**

(12) **EUROPEAN PATENT SPECIFICATION**

(45) Date of publication and mention
of the grant of the patent:
**09.03.2022 Bulletin 2022/10**

(21) Application number: **17739763.5**

(22) Date of filing: **16.06.2017**

(51) International Patent Classification (IPC):
**A61K 38/18** (2006.01)     **A61K 38/19** (2006.01)
**A61K 38/21** (2006.01)     **A61N 5/10** (2006.01)
**A61P 35/00** (2006.01)     **C07K 16/00** (2006.01)

(52) Cooperative Patent Classification (CPC):
**A61K 38/18; A61K 38/19; A61K 38/21;**
**A61P 35/00; C07K 16/2803;** A61K 2039/505;
A61N 2005/1098

(86) International application number:
**PCT/US2017/037998**

(87) International publication number:
**WO 2017/218970 (21.12.2017 Gazette 2017/51)**

(54) **IMMUNE MODULATORS IN COMBINATION WITH RADIATION TREATMENT**

IMMUNMODULATOREN IN KOMBINATION MIT EINER STRAHLENBEHANDLUNG

MODULATEURS IMMUNITAIRES EN COMBINAISON AVEC UN TRAITEMENT PAR
RAYONNEMENT

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **17.06.2016 US 201662351681 P**

(43) Date of publication of application:
**24.04.2019 Bulletin 2019/17**

(73) Proprietor: **Varian Medical Systems, Inc.**
**Palo Alto, CA 94304 (US)**

(72) Inventor: **PARRY, Renate**
**Palo Alto, California 94304 (US)**

(74) Representative: **Foster, Mark Charles**
**Mathisen & Macara LLP**
**Communications House**
**South Street**
**Staines-upon-Thames, Middlesex TW18 4PR**
**(GB)**

(56) References cited:
**WO-A1-2007/140236     WO-A2-2015/019284**
**US-A1- 2012 156 224     US-A1- 2015 118 222**
**US-A1- 2016 024 594**

- **MARKA CRITTENDEN ET AL: "Current Clinical Trials Testing Combinations of Immunotherapy and Radiation", SEMINARS IN RADIATION ONCOLOGY, vol. 25, no. 1, 1 January 2015 (2015-01-01), pages 54-64, XP055399097, US ISSN: 1053-4296, DOI: 10.1016/j.semradonc.2014.07.003**
- **DERER ANJA ET AL: "Immune-modulating properties of ionizing radiation: rationale for the treatment of cancer by combination radiotherapy and immune checkpoint inhibitors", CANCER IMMUNOLOGY, IMMUNOTHERAPY, SPRINGER, BERLIN/HEIDELBERG, vol. 65, no. 7, 21 November 2015 (2015-11-21), pages 779-786, XP035987447, ISSN: 0340-7004, DOI: 10.1007/S00262-015-1771-8 [retrieved on 2015-11-21]**
- **YUJO KAWASHITA ET AL: "An Autologous In Situ Tumor Vaccination Approach for Hepatocellular Carcinoma. 2. Tumor-Specific Immunity and Cure after Radio-Inducible Suicide Gene Therapy and Systemic CD40-Ligand and Flt3-Ligand Gene Therapy in an Orthotopic Tumor Model", RADIATION RESEARCH, vol. 182, no. 2, 1 August 2014 (2014-08-01), pages 201-210, XP055399231, US ISSN: 0033-7587, DOI: 10.1667/RR13617.1**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- M J WALTERS ET AL: "Inhibition of CXCR7 extends survival following irradiation of brain tumours in mice and rats", BRITISH JOURNAL OF CANCER, vol. 110, no. 5, 4 March 2014 (2014-03-04) , pages 1179-1188, XP055399240, GB ISSN: 0007-0920, DOI: 10.1038/bjc.2013.830
- G. K. PHILIPS ET AL: "Therapeutic uses of anti-PD-1 and anti-PD-L1 antibodies", INTERNATIONAL IMMUNOLOGY, vol. 27, no. 1, 16 October 2014 (2014-10-16), pages 39-46, XP055217958, ISSN: 0953-8178, DOI: 10.1093/intimm/dxu095

**Description**

**CROSS-REFERENCE TO RELATED APPLICATIONS**

**BACKGROUND OF THE INVENTION**

Radiation therapy is a key therapeutic modality for patients with cancer. Radiation can be delivered to the tumor with submillimeter precision while mostly sparing normal tissue, ultimately leading to tumor cell killing. However, the tumor cell's ability to escape the cell killing effects of radiation and/or to develop resistance mechanisms can counteract the tumor cell killing action of radiotherapy, potentially limiting the therapeutic effect of radiotherapy to treat cancer. Furthermore, the potential for normal tissue toxicity can impact the therapeutic window of radiation therapy as a treatment paradigm.

Radiation-induced tumor cell death leads to release of tumor antigens from lysed cells, increased MHC-1 expression on antigen presenting cells, and enhanced diversity of the intratumoral T-cell population. These factors and others are key to initiate activation of the body's own immune systems to eradicate cancer cells. Immune modulators are being explored to activate the body's own immune system, but are known to have limitations as monotherapy (*e.g.*, response rate in patients). The response rate of immune modulators when used as monotherapy is in the range of 20-30% of the targeted patient population. Combination approaches such as using two immune modulators or an immune modulator with a targeted anti-cancer drug have limitations due to systemic normal tissue toxicity.

**BRIEF SUMMARY OF THE INVENTION**

The present invention is defined by the claims and provides an immune modulator for use in a method of treating a tumor in a subject with cancer comprising administering an effective amount of ionizing radiation and the immune modulator to the tumor, according to claim 1. Optional features are defined in the dependent claims.

The methods described herein provide the dual benefits of anti-tumor efficacy and normal tissue protection when combining an immune modulator with ionizing radiation to treat cancer patients. Methods described herein can be used to treat local and metastatic cancers by administering ionizing radiation therapy to deliver a highly conformal dose to the tumor, and an immune modulator. This combination therapy has the potential to improve both the efficacy of radiation therapy both locally and systemically, and the efficacy of the immune modulators. The methods described herein also allow for the classification of patients into groups for receiving optimized radiation treatment based on patient specific biomarker signatures. The biomarker signature includes markers that have been shown to correlate with tumor agressiveness, radioresistance and poor prognosis.

In some aspects, provided herein is a method for treating a tumor in a subject with cancer comprising administering ionizing radiation and an immune modulator to the tumor. In some embodiments, the amount of ionizing radiation and immune modulator administered to the subject is effective at treating the tumor, for example, effective at killing one or more tumor cells, reducing the growth rate or size of the tumor, or eliminating the tumor from the body of the subject. Administration of the immune modulator was unexpectedly found to increase the anti-tumor response when combined with radiation therapy.

Provided herein are improved methods for treating a tumor that include administering an immune modulator and ionizing radiation to the subject with cancer. This combination therapy can elicit an increased anti-cancer response compared to immune modulator monotherapy or radiation monotherapy.

Other objects, features, and advantages of the present invention will be apparent to one of skill in the art from the following detailed description and figures.

**BRIEF DESCRIPTION OF THE DRAWINGS**

FIG. 1: Patient cohort treated at HFHS with either stereotactic body radiation therapy (SBRT) (12 Gy x 4) or conventional fractionated radiation (60 to 70 Gy).

FIG. 2 shows the role of CD44 and CD44-related signaling pathways (TGFβ pathway) in cancer. Modified from Thapa R, Wilson, GD: *Stem cells Int*, (2016).

FIG. 3A shows Allred IHC scoring, taking into account intensity and proportion of protein expression in cells. FIG. 3B shows expression levels of CD44 and MGF-E8 in lung tumor tissues.

FIG. 4 illustrates the role of TGFβ during radiation treatment.

FIGS. 5A, 5B, and 5C show that TGFβ activity in human NSCL histological subtypes correlates with radiation resistance. Immunostaining of ACD and SCC tumor samples is shown in FIG. 5A. FIG. 5B and 5C compare the level of TGFβ and activated SMAD2 in ACD and SCC samples.

FIG. 6 shows that combination treatment comprising an immune modulator and radiation can enhance inhibition of tumor growth compared to monotherapy.

FIG. 7 shows that combination treatment comprising an immune modulator and radiation can enhance inhibition of tumor growth compared to monotherapy.

FIGS. 8A-8E show TIM-4 expression in human lung tumor (FIG. 8A), colon tumor (FIG. 8B), prostate tumor (FIG. 8C) and breast tumor (FIG. 8D) and in a colon tumor bearing syngeneic C57/BL6 mouse model (FIG. 8E). FIG. 8F is the negative control.

FIGS. 9A-9D show MFGE-8 expressin in human lung tumor (FIG. 9A), human colon tumor (FIG. 9B), human prostate tumor (FIG. 9C), and human breast tumor (FIG. 9D).

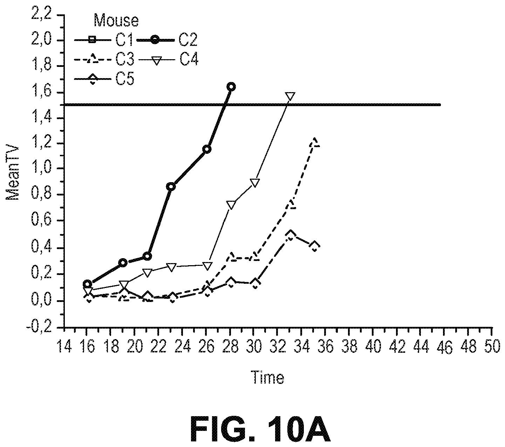

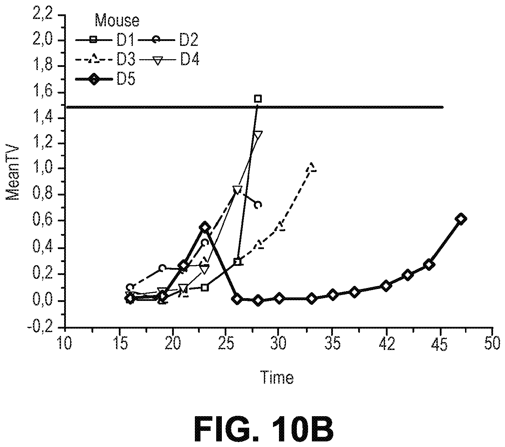

FIGS. 10A and 10B show that treatment comprising an immune modulator (anti-TIM-4 antibody) in combination with radiation can inhibit tumor growth compared to monotherapy with the immune modulator. Fig. 10A shows MC-38 carcinoma bearing mice were treated with anti-TIM4 antibody (2mg/kg) on days 17.19,21,23. Tumor volumes of individual mice (C1-C5) were monitored over the course of the treatment. Fig. 10B shows MC-38 carcinoma bearing mice were treated with radiation (2Gy) at day 16, followed by anti-TIM4 antibody administration (2mg/kg) on days 17.19,21,23. Tumor volumes of individual mice (D1-D5) wer monitored over the course of the treatment.

## DETAILED DESCRIPTION OF THE INVENTION

The present invention is defined by the claims. The methods described herein provide the advantages of anti-tumor efficacy and normal tissue protection when combining an immune modulator with ionizing radiation to treat cancer patients. The methods described herein provide the unexpected result that ionizing radiation in combination with immune modulator therapy can increase the anti-tumor response compared to treatment with radiation therapy or immune modulator therapy alone (monotherapy). The increase in the anti-tumor response can enhance or increase the inhibition of tumor growth that is provided by either monotherapy alone. Methods described herein can be used to treat local and metastatic cancers by administering ionizing radiation therapy to deliver a highly conformal dose to the tumor, and an immune modulator. The combination therapy described herein can improve both the efficacy of radiation therapy (locally and systemically) and the efficacy of the immune modulators. The immune modulator also enhances the anti-cancer response when administered in combination with radiation, compared to administration of either an immune modulator alone or radiation monotherapy.

## I. Definitions

The term "treating" refers to administering a treatment to a tumor or the subject diagnosed with a tumor. The treatment can be administered in an amount or therapeutic dose that is sufficient or effective to kill tumor cells, slow the growth of the tumor, reduce the size of the tumor, or eliminate the tumor from the subject entirely. Examples of treatments include ionizing radiation, an immune modulator agent, or a combination of both. The term also includes selecting a treatment or treatment plan, and providing treatment options to a healthcare provider or the subject.

The term "ionizing radiation" refers to radiation comprising particles having enough kinetic energy to discharge an electron from an atom or molecule, thereby producing an ion. The term includes both directly ionizing radiation, such as that caused by atomic particles such as alpha particles (helium nuclei), beta particles (electrons), and protons, and indirectly ionizing radiation, such as photons, including gamma rays and x-rays. Examples of ionizing radiation used in radiation therapy include high energy x-rays, electron beams, and proton beams.

The term "tumor environment" or "tumor micro-environment" refers to the immediate small-scale environment of an organism or part of an organism, especially as a distinct part of a larger environment, for example, the immediate small-scale environment of the tumor. The term includes not only the tumor cells themselves, but associated blood-vessels (including endothelial cells and smooth muscle cells), immune system cells and secreted cytokines, epithelial cells, fibroblasts, connective tissue, and/or extracellular matrix that is associated with or surrounds the tumor. The term also refers to the cellular and extracellular environment in which the tumor is located.

The term "standard of care" or "standard radiation treatment protocol" in radiation therapy generally refers to the ionizing radiation dose and administration interval that is generally accepted in the medical field as appropriate treatment for a given tumor, based on the tumor type, size, tissue location, and various other biological parameters. The standard of care or standard treatment protocol varies and is dependent on several factors. For example, for radiation therapy of lung cancer, the standard of care includes multiple fractions (e.g., approximately 30 fractions of low dose radiation, or approximately 60 Gy over 6 weeks) or a smaller number of fractions (e.g., 1-5 fractions) of biologically active doses (e.g., 54 GY in 3 fractions for peripheral tumors, or 48-60 Gy in 4-8 fractions for central tumors) administered to the tumor.

The term "similar dose of ionizing radiation" refers to a dose of ionizing radiation that is identical to, nearly the same, or substantially the same as the effective dose administered to a tumor in another subject, or administered to a tumor in the same subject undergoing an existing course of treatment. The term encompasses the normal and expected variation in ionizing radiation doses delivered by a medical technician skilled in the art of administering ionizing radiation to a tumor in a subject. For example, the term encompasses variation in the effective dose administered to a tumor of less than 10%, less than 5%, or less than 1%. The subject can be a human or non-human animal, such as a companion animal (e.g., cat, dog) or farm animal (*e.g.*, cow, horse, *etc.*).

The term "expression level" refers to the amount or level and/or the presence or absence of a biomarker described herein.

The term "small molecule drug" refers to an organic compound having a molecular weight of less than about 50 kDa, less than about 10 kDa, less than about 1 kDa, less than about 900 daltons, or less than about 500 daltons. The term includes drugs having desired pharmacological properties, and includes compounds that can be administered orally or by injection.

The term "radiosensitizer" refers to any substance that makes tumor cells easier to kill with radiation therapy. Exemplary radiosensitizers include hypoxia radiosensitizers such as misonidazole, metronidazole, and trans-sodium crocetinate, and DNA damage response inhibitors such as Poly (ADP) ribose polymerase (PARP) inhibitors.

The terms "sample," "biological sample," and "tumor sample" refer to bodily fluid, such as but not limited to blood, serum, plasma, or urine, and/or cells or tissues obtained from a subject or patient. In some embodiments, the sample is a formalin-fixed and paraffin embedded tissue or tumor sample. In some embodiments, the sample is a frozen tissue or tumor sample. In some embodiments, the tumor sample can be a biopsy comprising tumor cells from the tumor.

## II. Detailed Description

The present disclosure describes compounds for use in methods for treating a tumor in a subject by determining the expression levels of signature biomarkers in a tumor sample, comparing the expression levels in the tumor sample to the expression levels in a normal tissue sample, and treating the tumor if the expression levels in the tumor sample are different from those in the normal tissue sample. The treatment is ionizing radiation in combination with one or more immune modulators. Thus, the biomarkers provide so called "companion diagnostics" for the therapy to treat tumors. Methods described herein can be used to treat local and metastatic cancers by administering ionizing radiation therapy to deliver a highly conformal dose to the tumor, and an immune modulator.

The present disclosure comprising the invention describes an immune modulator used in combination with ionizing radiation in a method for treating a tumor in a subject with cancer comprising administering ionizing radiation and an immune modulator to the tumor . The immune modulator is a small molecule drug, or an antibody or a fragment thereof, that specifically binds to an inhibitory checkpoint molecule and inhibits the activity of the inhibitory checkpoint molecule and the inhibitory checkpoint molecule is TIM4 . In the embodiment of the invention, the inhibitor to the inhibitory checkpoint molecule is a small molecule drug, or an antibody or a fragment thereof that specifically binds to the inhibitory checkpoint molecule and inhibits its activity. In instances of the disclosure the inhibitor of MIF can be a small molecule drug, or antibody or fragment thereof that specifically binds to MIF and inhibits MIF activity. Other inhibitors of macrophage migration can also be used. In some instances of the disclosure, the immune modulator is an inhibitor of indoleamine 2, 3-dioxygenase (IDO).

The method can further include: (a) detecting an expression level of one or more biomarkers in a tumor sample from the subject, wherein the one or more biomarkers, e.g., 1, 2, 3, 4, 5 or more biomarkers are selected from the group consisting of an immune cell marker(s), tumor cell marker(s), circulating marker(s), and any combination thereof; (b) comparing the expression level of the one or more biomarkers, e.g., 1, 2, 3, 4, 5 or more biomarkers to the expression level of the one or more biomarkers, e.g., 1, 2, 3, 4, 5 or more biomarkers in a normal tissue sample; and (c) treating the tumor with ionizing radiation and an immune modulator if the expression level of the one or more biomarkers, e.g., 1, 2, 3, 4, 5 or more biomarkers is modified compared to the expression level in the normal tissue sample. In some instances, the expression level of the one or more biomarkers, e.g., 1, 2, 3, 4, 5 or more biomarkers is modified if the expression level of at least one of the biomarkers is increased, or the expression level of at least one of the biomarkers is decreased, or the expression level of at least one of the biomarkers is increased and the expression level of at least one of the biomarkers is decreased compared to the expression level in a normal tissue sample. The expression level of the one or more biomarkers, *e.g.,* 1, 2, 3, 4, 5 or more biomarkers can be ranked or weighted.

Optionally, the method further comprises performing functional imaging of the tumor prior to administering the ionizing radiation and the immune modulator.

In some instances of the disclosure, the immune cell biomarker(s) or the tumor cell biomarker(s) or the circulating biomarker(s) is a polynucleotide or a protein. The step of detecting can be performed by using an assay selected from the group consisting of immunohistochemistry, ELISA, Western analysis, HPLC, proteomics, PCR, RT-PCR, Northern analysis, and a microarray.

The tumor sample can be a biopsy comprising tumor cells. The normal tissue sample can comprise non-tumor cells from the same tissue type as the tumor.

The ionizing radiation is administered at a higher dose compared to a standard treatment protocol if the expression level of the one or more biomarkers, e.g., 1, 2, 3, 4, 5 or more biomarkers in the tumor sample is modified compared to the expression level in the normal tissue sample. In certain instances, the ionizing radiation is administered as a hypofractionated radiation treatment if the expression level of the one or more biomarkers, e.g., 1, 2, 3, 4, 5 or more biomarkers in the tumor sample is modified compared to the expression level in the normal tissue sample. In other instances, the ionizing radiation is administered as a hyperfractionated radiation treatment if the expression level of the one or more biomarkers, e.g., 1, 2, 3, 4, 5 or more biomarkers in the tumor sample is modified compared to the expression level in the normal tissue sample.

The ionizing radiation and the immune modulator can be administered concomitantly. Alternatively, the ionizing radiation and the immune modulator can be administered sequentially.

In another instance of the disclosure, provided herein is a method of treating a tumor in a subject with cancer comprising: (a) determining an expression level of one or more biomarkers, e.g., 1, 2, 3, 4, 5 or more biomarkers in a tumor sample from the subject, wherein the one or more biomarkers, e.g., 1, 2, 3, 4, 5 or more biomarkers are selected from the group consisting of an immune cell marker(s), tumor cell marker(s), circulating marker(s), and any combination thereof; (b) comparing the expression level of the one or more biomarkers, e.g., 1, 2, 3, 4, 5 or more biomarkers to an expression level of the one or more biomarkers, e.g., 1, 2, 3, 4, 5 or more biomarkers in a normal tissue sample; and (c) administering to the tumor in the subject a treatment comprising ionizing radiation and an immune modulator if the expression level of the one or more biomarkers, e.g., 1, 2, 3, 4, 5 or more biomarkers in the tumor sample is modified compared to the expression level in the normal tissue sample.

In some instances of the disclosure, the expression level of the one or more biomarkers, e.g., 1, 2, 3, 4, 5 or more biomarkers is modified if the expression level of at least one of the biomarkers is increased, or the expression level of at least one of the biomarkers is decreased, or the expression level of at least one of the biomarkers is increased and the expression level of at least one of the biomarkers is decreased compared to the expression level in a normal tissue sample. The expression level of the one or more biomarkers, e.g., 1, 2, 3, 4, 5 or more biomarkers can be ranked or weighted.

In some instances of the disclosure, the step of administering ionizing radiation comprises contacting the tumor with a radiosensitizer. The ionizing radiation can be administered at a higher dose compared to a standard treatment protocol if the expression level of the one or more biomarkers, e.g., 1, 2, 3, 4, 5 or more biomarkers in the tumor sample is modified compared to the expression level in the normal tissue sample. The ionizing radiation can be administered as a hypofractionated radiation treatment if the expression level of the one or more biomarkers, e.g., 1, 2, 3, 4, 5 or more biomarkers in the tumor sample is modified compared to the expression level in the normal tissue sample. In other cases, the ionizing radiation is administered as a hyperfractionated radiation treatment if the expression level of the one or more biomarkers, e.g., 1, 2, 3, 4, 5 or more biomarkers in the tumor sample is modified compared to the expression level in the normal tissue sample.

The immune modulator can be selected from the group consisting of an inhibitor to an inhibitory checkpoint molecule, an activator of a stimulatory checkpoint molecule, a chemokine inhibitor, an inhibitor of macrophage migration inhibitory factor (MIF), a growth factor, a cytokine, an interleukin, an interferon, an antibody that binds to an immune system cell, a cellular immune modulator, a vaccine, an oncolytic virus, and any combination thereof. The ionizing radiation and the immune modulator are administered concomitantly. In certain instances, the ionizing radiation and the immune modulator are administered sequentially.

The method described herein can also include performing functional imaging of the tumor prior to administering the ionizing radiation and the immune modulator.

In yet another instance of the disclosure, provided herein is a method of identifying a subject with cancer as a candidate for treatment comprising ionizing radiation and an immune modulator. The method comprises (a) determining an expression level of one or more biomarkers, e.g., 1, 2, 3, 4, 5 or more biomarkers in a tumor sample from the subject, wherein the one or more biomarkers are selected from the group consisting of an immune cell marker(s), tumor cell marker(s), circulating marker(s), imaging marker(s), and any combination thereof; (b) comparing the expression level of the one or more biomarkers, e.g., 1, 2, 3, 4, 5 or more biomarkers to an expression level of the one or more biomarkers, e.g., 1, 2, 3, 4, 5 or more biomarkers in a normal tissue sample; and (c) classifying the subject as a candidate for treatment comprising ionizing radiation and the immune modulator if the expression level of the one or more biomarkers, *e.g.*, 1, 2, 3, 4, 5 or more biomarkers in the tumor sample is modified compared to the expression level in the normal tissue sample. In some instances, the expression level of the one or more biomarkers, e.g., 1, 2, 3, 4, 5 or more biomarkers is modified if the expression level of at least one of the biomarkers is increased, or the expression level of at least one of the biomarkers is decreased, or the expression level of at least one of the biomarkers is increased and the expression level of at least one of the biomarkers is decreased compared to the expression level in a normal tissue sample. In certain cases, the expression level of the one or more biomarkers, e.g., 1, 2, 3, 4, 5 or more biomarkers is ranked or weighted. In some cases, the method further comprises performing functional imaging of the tumor.

In some instances of the disclosure, the immune modulator is selected from the group consisting of an inhibitor to an inhibitory checkpoint molecule, an activator of a stimulatory checkpoint molecule, a chemokine inhibitor, an inhibitor of macrophage migration inhibitory factor (MIF), a growth factor, a cytokine, an interleukin, an interferon, an antibody that binds to an immune system cell, a cellular immune modulator, a vaccine, an oncolytic virus, and any combination thereof. The ionizing radiation can be administered at a higher dose compared to a standard treatment protocol if the expression level of the one or more biomarkers, e.g., 1, 2, 3, 4, 5 or more biomarkers in the tumor sample is modified compared to the expression level in the normal tissue sample. In some instances, the ionizing radiation is administered as a hypofractionated radiation treatment if the expression level of the one or more biomarkers, e.g., 1, 2, 3, 4, 5 or more biomarkers in the tumor sample is modified compared to the expression level in the normal tissue sample. In other instances, the ionizing radiation is administered as a hyperfractionated radiation treatment if the expression level of the one or more biomarkers, e.g., 1, 2, 3, 4, 5 or more biomarkers in the tumor sample is modified compared to the expression level in the normal tissue sample. The ionizing radiation and the immune modulator are administered concomitantly. The ionizing radiation and the immune modulator are administered sequentially.

In another instance of the disclosure, provided herein is a method of selecting a treatment for a subject with cancer comprising (a) determining an expression level of one or more biomarkers, e.g., 1, 2, 3, 4, 5 or more biomarkers in a tumor sample from the subject, wherein the one or more biomarkers are selected from the group consisting of an immune cell marker(s), tumor cell marker(s), circulating marker(s), and any combination thereof; (b) comparing the expression level of the one or more biomarkers, e.g., 1, 2, 3, 4, 5 or more biomarkers to an expression level of the one or more biomarkers, e.g., 1, 2, 3, 4, 5 or more biomarkers in a normal tissue sample; and (c) selecting a treatment comprising ionizing radiation and an immune modulator if the expression level of the one or more biomarkers, e.g., 1, 2, 3, 4, 5 or more biomarkers in the tumor sample is modified compared to the expression level in the normal tissue sample. In some embodiments, comprising performing functional imaging of the tumor; and selecting the treatment comprising the ionizing radiation and the immune modulator based on the functional imaging of the tumor. In some cases, the ionizing radiation comprises contacting the tumor with a radiosensitizer.

In some embodiments, the expression level of the one or more biomarkers, e.g., 1, 2, 3, 4, 5 or more biomarkers is modified if the expression level of at least one of the biomarkers is increased, or the expression level of at least one of the biomarkers is decreased, or the expression level of at least one of the biomarkers is increased and the expression level of at least one of the biomarkers is decreased compared to the expression level in a normal tissue sample. The expression level of the one or more biomarkers, *e.g.*, 1, 2, 3, 4, 5 or more biomarkers can be ranked or weighted.

In some instances of the disclosure, the immune modulator is selected from the group consisting of an inhibitor to an inhibitory checkpoint molecule, an activator of a stimulatory checkpoint molecule, a chemokine inhibitor, an inhibitor of macrophage migration inhibitory factor (MIF), a growth factor, a cytokine, an interleukin, an interferon, an antibody that binds to an immune system cell, a cellular immune modulator, a vaccine, an oncolytic virus, and any combination thereof. The ionizing radiation can be administered at a higher dose compared to a standard treatment protocol if the expression level of the one or more biomarkers in the tumor sample is modified compared to the expression level in the normal tissue sample. In some cases, the ionizing radiation is administered as a hypofractionated radiation treatment if the expression level of the two or more biomarkers in the tumor sample is modified compared to the expression level in the normal tissue sample. In other cases, the ionizing radiation is administered as a hyperfractionated radiation treatment if the expression level of the one or more biomarkers in the tumor sample is modified compared to the expression level in the normal tissue sample.

In another instance of the disclosure, a kit is provided. The kit comprises reagents capable of detecting expression of the biomarkers described herein. In some instances of the disclosure, the kit comprises reagents capable of detecting nucleic acid *(e.g.,* RNA) expression of the biomarkers. For example, the kit can comprise oligonucleotide primers that are capable amplifying a nucleic acid expressed by the biomarker genes described herein. In some instances of the disclosure, the kit further comprises an oligonucleotide probe that hybridizes to a biomarker nucleic acid or an amplified biomarker nucleic acid, or a complement thereof. Methods of amplifying and detecting nucleic acids are well known in the art, and can comprise PCR, RT-PCR real-time PCR, and quantitative real-time PCR, Northern analysis, sequencing of expressed nucleic acids, and hybridization of expressed and/or amplified nucleic acids to microarrays. In some instances of the disclosure, the kit comprises reagents that are capable of detecting proteins expression by the biomarkers described herein. In some instances of the disclosure, the reagents are antibodies that specifically bind to biomarker proteins. Methods of detecting protein expression are well known in the art, and include immunoassays, ELISA, Western analysis, and proteomic techniques.

In some instances of the disclosure, the differences in the expression levels of each of the biomarkers in the tumor sample are increased or decreased by at least 10%, 20%, 30%, 40%, 50%, 60%, 70%, 80%, 90% or more compared to the expression level in normal tissue. In some instances of the disclosure, the expression levels of each of the biomarkers in the tumor sample are increased or decreased by at least 1-fold, 2-fold, 3-fold, 4-fold, 5-fold, 6-fold, 7-fold, 8-fold, 9-fold, 10 fold or more relative to the expression level in normal tissue.

In some instances of the disclosure, the average and/or ranked expression level of all the biomarkers in the tumor sample is increased or decreased relative to the expression level in normal tissue. Thus, in some instances of the disclosure, the average and/or ranked expression level of all the biomarkers in the tumor sample is increased or decreased by at least 10%, 20%, 30%, 40%, 50%, 60%, 70%, 80%, 90% or more compared to the expression level in normal tissue. In some instances of the disclosure, the expression levels in normal tissue are normalized to a control or baseline level. It will be understood that the expression level can also be compared to the expression level in the tumor sample before, after or during a treatment, course of treatment, or treatment plan. Thus, in some instances of the disclosure, the expression levels of each of the biomarkers in the tumor sample are increased or decreased by at least 10%, 20%, 30%, 40%, 50%, 60%, 70%, 80%, 90% or more compared to the expression level in the tumor sample before, during or after treatment.

Further, with regard to any of the above instances of the disclosure, the one or more biomarkers can comprise or consist of any combination of the biomarkers, for example, any of the biomarkers described herein, any combination of two or more biomarkers, any combination of three or more biomarkers, any combination of four or more biomarkers, any combination of five or more biomarkers, any combination of six or more biomarkers, and any combination of seven or more biomarkers.

In another instance of the disclosure, the expression level of at least one, two, three, four or more of the biomarkers described herein is determined. The combination of expression levels of two or more biomarkers, e.g., 2, 3, 4, 5, 6 or more biomarkers can indicate that the subject with cancer is more sensitive to radiation compared to a control subject. This subject may be administered a reduced or decreased dose of radiation compared to a standard dose. In other instances, if the combination of expression levels of two or more biomarkers, e.g., 2, 3, 4, 5, 6 or more biomarkers can indicate that the subject with cancer is less sensitive to radiation compared to a control subject. A subject who is less sensitive to radiation may be administered an increased dose, a hypofractionated dose or a hyperfractionated dose of radiation.

In some embodiments, the biomarker is CD44, MFG-E8, CD68, TGFβ, or any combination thereof. In certain instances of the disclosure, if a first biomarker has a high level of expression and a second biomarker has a low level of expression in a sample obtained from a subject with cancer relative to a control sample, then it is predicted that radiation treatment monotherapy may result in local tumor control failure. As such, this biomarker profile can indicate that the subject should be administered radiation treatment in combination with an immune modulator. Alternatively, this biomarker profile can indicate that the dose of radiation be increased (i.e., increased over a standard protocol dose). For instance, if the level of CD44 is high and the level of MFG-E8 is low in a subject's tumor sample compared to a control sample, then it is predicted that radiation treatment alone will not lead to a clinical response. In other words, a tumor sample having a high level of CD44 and a low level of MFG-E8 is likely to be insensitive or have a low sensitivity to ionizing radiation therapy. In some cases, the biomarker profile described herein indicates that the subject should receive an increased dose of radiation and/or combination therapy comprising ionizing radiation and an immune modulator, such as an anti-TIM4 antibody, anti-MFG-E8 antibody, anti-M199 antibody, and any combination thereof.

In other instances of the disclosure, if the level of CD44 is low compared to a normal sample and/or the level of MFG-E8 is high compared to a normal sample, the subject is likely to have a clinical response to ionizing radiation monotherapy. In some cases, it is predicted that a subject with low level of CD44 and/or a high level of MFG-E8 is likely to be sensitive to ionizing radiation therapy.

In some instances of the disclosure, if a subject's tumor has a high level of CD68 compared to a control sample, the subject is predicted to have decreased survival after radiation monotherapy. As such, this subject can be administered a combination therapy comprising ionizing radiation and an immune modulator. In other instances, if a subject's tumor has a low level of CD68 compared to a control sample, the subject is likely to have a clinical response to radiation monotherapy. It is predicted that this subject is sensitive to radiation. In certain cases, it may be indicated that the subject be administered a low dose or reduced dose of radiation compared to a standard protocol dose.

A. Biomarkers for Therapy Selection

The biomarkers described herein can be used to stratify patients to receive individualized, tailored radiotherapy in combination with an immune modulator agent. The biomarkers can also be used to monitor the efficacy of immune modulator therapy on patients with cancer. The biomarkers include, but are not limited to, one or more immune cell biomarkers, one or more tumor cell biomarkers, one or more circulating biomarkers, one or more imaging biomarkers, and any combination thereof. For instances, an immune cell biomarker can provide information about the location and/or activity of a specific cell population, such as a T cell population. An immune cell biomarker or tumor cell biomarker can be a genetic biomarker, polynucleotide biomarker, or a protein biomarker. In some embodiments, an immune cell biomarker is a specific polynucleotide (e.g., RNA and microRNA) or protein that is expressed at a higher level by a particular immune cell compared to a non-immune cell or a different type of immune cell. Similarly, a tumor cell biomarker can a specific polynucleotide (e.g., RNA and microRNA) or protein that is expressed at a higher level by a tumor cell compared to a non-tumor cell. For example, the tumor cell biomarker can be a protein or a polynucleotide encoding said protein that is associated with proliferation and/or metastasis of a tumor cell. In some cases, the protein can be involved in angiogenesis or other processes that are activated by a tumor cell. The tumor biomarker can be an oncogene or a tumor suppressor. In some instances, a tumor cell biomarker is a gene variation, gene mutation, copy number variant (CNV), single nucleotide polymorphism (SNP), and the like that is present in a tumor cell, but not in a non-tumor cell. In some instances of the disclosure, a circulating biomarker is an exosome (*i.e.*, a cell-derived vesicle that can be found in a body fluid). Examples of useful biomarkers includes those described in U.S. Patent Appl. Publ. No. 20160024594.

The biomarker set can include, but is not limited to, CD44, milk fat globule-EGF factor 8 (MFG-E8), CD68 and TGFβ. CD44 is a cell-surface glycoprotein that plays a role in cell proliferation, cell-cell interactions, cell adhesion, and cell migration of various cell types including lymphocytes and cancer cells. The human CD44 polypeptide sequence is set forth in, e.g., GenBank Accession No. NP_000601. The human CD44 mRNA (coding) sequence is set forth in, e.g., GenBank Accession No. NM 000610. Milk fat globule-EGF factor 8 protein (MFG-E8) is a macrophage-produced protein that promotes engulfment and clearance of apoptotic cells in tumors. The human MFG-E8 polypeptide sequence is set forth in, *e.g.*, GenBank Accession No. NP_005919. The human MFG-E8 mRNA (coding) sequence is set forth in, e.g., GenBank Accession No. NM_005928. CD68 is a 110-kD transmembrane glycoprotein that is highly expressed by human monocytes and tissue macrophages. The protein primarily localizes to lysosomes and endosomes with a smaller fraction circulating to the cell surface. It is a type I integral membrane protein with a heavily glycosylated extracellular domain and binds to tissue- and organ-specific lectins or selectins. CD68 is also a member of the scavenger receptor family. The human CD68 polypeptide sequence is set forth in, e.g., GenBank Accession No. NP_001242. The human CD68 mRNA (coding) sequence is set forth in, *e.g.*, GenBank Accession No. NM_001251. TGFβ is a cytokine that is involved in cell growth, cell proliferation, cell differentiation, apoptosis, homeostasis and many other cellular processes. The human TGFβ polypeptide sequence is set forth in, e.g., GenBank Accession No. NP_000651. The human TGFβ mRNA (coding) sequence is set forth in, e.g., GenBank Accession No. NM_000660.

It will be understood that the expression levels of each of the biomarkers described herein in the patient sample can increase or decrease relative to the expression level of the tumor biomarker in a normal or control tissue sample. For example, the expression level of one tumor biomarker can increase in the tumor sample compared to the expression level in a normal tissue, whereas the expression level of a second biomarker can decrease in the tumor sample compared to the expression level in a normal tissue. The expression level can also be based on the average, combination or sum of the all the tumor biomarker expression levels in the patient sample. For example, the expression level of each biomarker in the patient sample can be ranked or weighted to produce a ranked value that is higher or lower than the normal tissue value (which can be a normalized value, for example, set to 1).

In some embodiments, biomarker expression is determined in a biological sample from the subject having a tumor. In some embodiments, the biological sample is a tumor sample. The tumor sample can be a biopsy comprising tumor cells from the tumor. In some embodiments, the biological sample comprises a bodily fluid, such as but not limited to blood, serum, plasma, or urine, and/or cells or tissues from the subject. In some embodiments, the biological sample is a formalin-fixed and paraffin embedded tissue or tumor sample. In some embodiments, the biological sample is a frozen tissue or tumor sample. Thus, in some embodiments, one or more steps of the methods described herein are carried out *in vitro.* For example, in some embodiments, biomarker expression is determined *in vitro.*

In some instances of the disclosure, the normal tissue sample comprises non-tumor cells from the same tissue type as the tumor. In some instances of the disclosure, the normal tissue sample is obtained from the same subject diagnosed with the tumor. A normal tissue sample can also be a control sample of the same tissue-type from a different subject. The expression level of the normal tissue sample can also be an average or mean value obtained from a population of normal tissue samples.

The level of expression of the biomarkers described herein can be determined using any method known in the art. For example, the level of expression can be determined by detecting the expression of a nucleic acid (e.g., RNA, mRNA or microRNA) or the protein encoded by the nucleic acid.

Exemplary methods for detecting expression levels of nucleic acids include, without limitation, Northern analysis, polymerase chain reaction (PCR), reverse transcription PCR (RT-PCR), real-time PCR, quantitative real-time PCR, and DNA microarrays.

Exemplary methods for detecting expression levels of proteins (e.g., polypeptides) include, without limitation, immunohistochemistry, ELISA, Western analysis, HPLC, and proteomics assays. In some instances of the disclosure, the protein expression level is determined by immunohistochemistry using the Allred method to assign a score (*see,* *e.g.*, Allred, D. C., Connection 9:4-5, 2005). For example, formalin-fixed, paraffin embedded tissues are contacted with an antibody that specifically binds a biomarker described herein. The bound antibody is detected with a detectable label or secondary antibody coupled with a detectable label, such as a colorimetric label (e.g., an enzymatic substrate produce by HRP or AP). The antibody positive signal is scored by estimating the proportion of positive tumor cells and their average staining intensity. Both the proportion and intensity scores are combined into a total score that weighs both factors.

In some instances of the disclosure, the protein expression level is determined by digital pathology. Digital pathology methods include scanning images of tissues on a solid support, such as a glass slide. The glass slides are scanned into whole slide images using a scanning device. The scanned images are typically stored in an information management system for archival and retrieval. Image analysis tools can be used to obtain objective quantitative measurements from the digital slides. For example, the area and intensity of immunohistochemical staining can be analyzed using the appropriate image analysis tools. Digital pathology systems can include scanners, analytics (visualization software, information management systems and image analysis platforms), storage and communication (sharing services, software). Digital pathology systems are available from numerous commercial suppliers, for example. Aperio Technologies, Inc. (a subsidiary of Leica Microsystems GmbH), and Ventana Medical Systems, Inc. (now part of Roche). Expression levels can be quantified by commercial service providers, including Flagship Biosciences (CO), Pathology, Inc. (CA), Quest Diagnostics (NJ), and Premier Laboratory LLC (CO).

In some instances of the disclosure, imaging of the tumor, such as functional imaging is also used to identify or select a cancer patient who should receive the combination therapy described herein. Non-limiting examples of functional imaging include single-photon emission computed tomography, optical imaging, ultrasonography, positron emission tomography (PET), computed tomography (CT), perfusion computed tomography, magnetic resonance imaging (MRI), functional magnetic resonance imaging, magnetic resonance sectroscopic imaging, dynamic contrast-enhanced imaging, diffusion-weighted imaging, blood-oxygenation level dependent imaging, magnetic resonance spectroscopy, magnetic resonance lymphography, and any combination thereof. Any type of functional imaging such as multimodality imaging can be performed to characterize the tumor, to determine the delineation of the tumor, the extent of the tumor, the tumor volume, and/or to assess the tumor microenvironment (e.g., the environment surrounding the tumor). Functional imaging can aid in selecting the best treatment option and/or in monitoring response to the treatment.

## B. Methods for Selecting a Course of Treatment

The expression levels of the biomarkers can be used to determine or select a course of treatment in a subject diagnosed with a tumor. The treatment comprises adminstering ionizing radiation to the tumor in the subject. The ionizing radiation can also be administered to the entire subject or a portion thereof, especially if the tumor is dispersed or mobile. In some instances of the disclosure, the treatment further comprises contacting the tumor with a radiosensitizer. The treatment further comprises administering a compound or biologic drug, such as an antibody, that inhibits an immune checkpoint pathway to the subject. Thus, in some embodiments, the treatment comprises administering a standard radiation treatment protocol in combination with an immune modulator.

The course of treatment can be selected based on the expression levels of the biomarkers. For example, the expression levels can be used to determine if radiation therapy is appropriate for the subject (*i.e.,* for making a go/no go decision on radiotherapy). Further, if the expression levels of the biomarkers are increased relative to a normal or control value, then the effective radiation dose to the tumor can be increased, and/or the fractionation schedule modified accordingly. The radiation dose to the blood vessels feeding the tumor can also be increased. In some cases, a hypofractionated radiation treatment is administered. Alternatively, a hyperfractionated radiation treatment is administered. Optionally, radiation treatment is provided in combination with immune modulator treatment.

In some embodiments, if the expression levels of the biomarkers are increased relative to a normal or control value, then the treatment can comprise administering ionizing radiation to the tumor. In some embodiments, if the expression levels of the biomarkers are decreased relative to a normal or control value, then the treatment can comprise decreasing the amount of ionizing radiation administered to the tumor.

The treatment can also comprise modifying an existing course of treatment. For example, in some embodiments, the existing course of treatment is modified to increase the effective dose of the ionizing radiation administered to the tumor. In some instances of the disclosure, the effective dose of ionizing radiation is increased by increasing the amount of ionizing radiation administered to the tumor and/or contacting the tumor with a radiosensitizer. In some embodiments, the existing course of treatment is modified to decrease the effective dose of the ionizing radiation administered to the tumor. In some embodiments, the treatment comprises modifying a standard radiation treatment protocol in combination with administering an immune modulator.

In some embodiments, the effective dose of ionizing radiation administered to the tumor is increased if the level of one or more biomarkers described herein is elevated in the tumor environment. For example, the effective dose of ionizing radiation is increased as compared to the standard of care for a subject that does not have elevated levels of the biomarker(s) in the tumor environment. This applies to subjects who are currently not undergoing radiation therapy as well as modifying an existing course of treatment for subjects undergoing radiation therapy. Thus, the effective dose of ionizing radiation can be increased from the current effective dose if the subject is already undergoing radiation therapy for a tumor. The radiation therapy can be modified to reduce the constraints on neighboring healthy tissue. For example, if the biomarker level in the tumor environment indicates more aggressive radiation therapy is required, the treatment plan can be modified so that the constraints on the border between healthy tissue and tumor tissue are decreased. This would result in a trade-off between damaging some healthy tissue in order to kill more of the tumor tissue.

In some instances of the disclosure, the treatment comprises a combination of radiation therapy and an immune modulator agent (including a radiosensitizer). In some embodiments, the effective dose of ionizing radiation administered to the tumor is not changed (e.g., relative to the standard of care or relative to an existing course of treatment) when an immune modulator agent is administered to the subject. For example, in some embodiments, the subject is administered an effective dose of ionizing radiation that is the same or similar to that administered to a subject that does not have elevated levels of one or more biomarkers described herein in the tumor environment, and the subject is further administered an immune modulator agent. In some embodiments, the effective dose of ionizing radiation administered to the tumor is based on the standard of care for a subject that does not have elevated levels of the biomarker(s) in the tumor environment, and the subject is further administered an immune modulator agent. In some embodiments involving an existing course of treatment, the effective dose of ionizing radiation is maintained at the current effective dose, and an anti-cancer agent is administered to the subject in combination with the ionizing radiation if the level of one or more biomarkers described herein is elevated in the tumor environment.

In some embodiments, the treatment plan is developed and/or modified based on the expression levels of the biomarkers described herein.

The course of treatment can also be selected by using an algorithm that determines the expression level of the biomarkers in the tumor sample relative to the level in the normal sample. The algorithm can be a linear regression algorithm that includes the biomarker expression levels and coeffcients (i.e., weights) for combining the expression levels. In some instances of the disclosure, the algorithm comprises a least squares fit to calculate the coefficients. If the algorithm determines that the expression level of the biomarkers in the tumor sample is increased or decreased relative to the normal sample, then the appropriate course of treatment can be assigned. In some instances of the disclosure, the algorithm is a nonparametric regression tree. In some instances of the disclosure, standard statistical methods were used to analyze the data to determine which biomarkers were most predictive of clinical survival or local tumor control failure.

In some instances of the disclosure, the method described herein is a computer implemented method. In some instances of the disclosure, the computer implemented method comprises a linear regression model that assigns a ranked or weighted value to the expression levels of the biomarkers desribed herein. In some instances of the disclosure, the disclosure provides a computer-readable medium, the medium providing instructions to cause a computer to perform a method described herein. For example, the medium can provide instructions to cause a computer to assign a ranked or weighted value to the expression levels of the biomarkers desribed herein.

## C. Radiation Therapy

The expression levels of the tumor biomarkers described herein can be used to optimize treatment of patients with radiotherapy. For example, the therapeutic dose of the radiation adminstered to the tumor or subject can be adjusted based on the expression levels of the biomarkers. As is well known in the art, the effective dose of ionizing radiation varies with the type of tumor and stage of cancer that needs to be treated. The effective dose can also vary based on other treatment modalities being administered to the patient, for example chemotherapeutic treatments and surgical treatments, and whether the radiation is administered pre- or post-surgery. In general, a curative therapeutic dose for a solid epithelial tumor ranges from about 60 to 80 gray (Gy), whereas a curative dose for a lymphoma is about 20 to 40 Gy. In general, preventative doses can be 45-60 Gy.

As is well known in the art, the therapeutic dose can be delivered in fractions. Fractionation refers to spreading out the total dose of radiation over time, for example, over days, weeks or months. The dose delivered in each fraction can be about 1.5-2 Gy per day. The treatment plan can include a fraction treatment one or more times per day, every other day, weekly, etc. depending on the treatment needs of each patient. For example, a hypofractionation schedule comprises dividing the total dose into several relativley large doses, and administering the doses at least one day apart. Exemplary hypofraction doses are 3 Gy to 20 Gy per fraction. An exemplary fractionation schedule that can be used to treat lung cancer is Continuous Hyperfractionated Accelerated Radiation therapy (CHART), which consists of three small fractions per day.

In some instances of the disclosure, the ionizing radiation includes contacting the tumor in the subject with a radiosensitizer. Exemplary radiosensitizers include hypoxia radiosensitizers such as misonidazole, metronidazole, and trans-sodium crocetinate, a compound that helps to increase the diffusion of oxygen into hypoxic tumor tissue. The radiosensitizer can also be a DNA damage response inhibitor interfering with base excision repair (BER), nucleotide excision repair (NER), mismatch repair (MMR), recombinational repair comprising homologous recombination (HR) and non-homologous end-joining (NHEJ), and direct repair mechanisms. SSB repair mechanisms include BER, NER, or MMR pathways whilst DSB repair mechanisms consist of HR and NHEJ pathways. Radiation causes DNA breaks that if not repaired are lethal. Single strand breaks are repaired through a combination of BER, NER and MMR mechanisms using the intact DNA strand as a template. The predominant pathway of SSB repair is the BER utilizing a family of related enzymes termed poly-(ADP-ribose) polymerases (PARP). Thus, the radiosensitizer can include DNA damage response inhibitiors such as Poly (ADP) ribose polymerase (PARP) inhibitors.

The biomarkers described herein are useful in developing and modifying treatment plans for patients diagnosed with a tumor or cancer. The treatment plan can include visualizing or measuring the tumor volume that needs to be irradiated, the optimal or effective dose of radiation administered to the tumor, and the maximum dose to prevent damage to nearby healthy tissue or organs at risk. Algorithms can used in treatment planning, and include dose calculation algorithms based on the particular radiotherapy technique parameters employed, e.g., gantry angle, MLC leaf positions, *etc.*, and search algorithms which use various techniques to adjust system parameters between dose calculations to optimize the effectiveness of the treatment. Exemplary dose calculation algorithms include various Monte Carlo ("MC") techniques and pencil beam convolution ("PBC"). Exemplary search algorithms include various simulated annealing ("SA") techniques, algebraic inverse treatment planning ("AITP"), and simultaneous iterative inverse treatment planning ("SIITP"). Such techniques, and others, are well known in the art, and are included within the scope of this disclosure.

Treatment planning algorithms may be implemented as part of an integrated treatment planning software package which provides additional features and capabilities. For example, a dose calculation algorithm and search algorithm may be used to optimize a set of fluence maps at each gantry angle, with a separate leaf sequencer used to calculate the leaf movements needed to deliver them. Alternatively, a dose calculation algorithm and search algorithm may be used to directly optimize leaf movements and other machine parameters. The Eclipse™ Treatment Planning System offered by the assignee of the present invention includes such an integrated software program. Methods for optimizing treatment plans are described in U.S. Patent No. 7,801,270.

In some embodiments, the biomarkers described herein can be used to monitor the progress of tumor control after radiation therapy. For example, the expression levels of the biomarkers before and after ionizing radiation therapy can be compared. In some embodiments, if the expression levels of biomarkers increase after radiotherapy, this suggests that the tumor is continuing to grow in size. Thus, the radiation treatment can be modified based on monitoring tumor growth using the biomarkers described herein.

The biomarkers described herein can be used with any radiation therapy technique known in the art. Radiation therapy techniques include external-beam radiotherapy ("EBRT") and Intensity Modulated Radiotherapy ("IMRT"), which can be administered by a radiotherapy system, such as a linear accelerator, equipped with a multileaf collimator ("MLC"). The use of multileaf collimators and IMRT allows the patient to be treated from multiple angles while varying the shape and dose of the radiation beam, thereby avoiding excess irradiation of nearby healthy tissue. Other exemplary radiation therapy techniques include stereotactic body radiotherapy (SBRT), volumetric modulated arc therapy, three-dimensional conformal radiotherapy ("3D conformal" or "3DCRT"), image-guided radiotherapy (IGRT). The radiation therapy techniques can also include Adaptive radiotherapy (ART), a form of IGRT that can revise the treatment during the course of radiotherapy in order to optimize the dose distribution depending on patient anatomy changes, and organ and tumor shape. Another radiation therapy technique is brachytherapy. In brachytherapy, a radioactive source is implanted within the body of the subject, such that the radioactive source is near the tumor. As used herein, the term radiotherapy should be broadly construed and is intended to include various techniques used to irradiate a patient, including use of photons (such as high energy x-rays and gamma rays), particles (such as electron and proton beams), and radiosurgical techniques. Further, any method of providing conformal radiation to a target volume is intended to be within the scope of the present disclosure.

### D. Immune Modulators

The radiation therapy is administered in combination with one or more immune modulators. The combination therapy can provide an increased anti-tumor response (a positive clinical response) compared to administration of either treatment as monotherapy. In some instances of the disclosure, the immune modulator can be selected from the group consisting of an inhibitor to an inhibitory checkpoint molecule, an activator of a stimulatory checkpoint molecule, a chemokine inhibitor, an inhibitor of macrophage migration inhibitory factor (MIF), a growth factor, a cytokine, an interleukin, an interferon, an antibody that binds to an immune system cell, such as a bispecific antibody that binds to T-cells and a tumor antigen, a cellular immune modulator such as a CAR-T cell, a vaccine, an oncolytic virus, and any combination thereof.

According to the invention the immune modulator is a small molecule drug, or an antibody or a fragment thereof, that specifically binds to an inhibitory checkpoint molecule and inhibits the activity of the inhibitory checkpoint molecule and the inhibitory checkpoint molecule is TIM4. Immune modulators can include small molecules and biologic therapies (e.g., antibodies, fragments thereof, and derivatives thereof) that bind molecules expressed on the surface of immune system cells, such as antigen presenting cells and T-cells. Immune modulators also can include small molecules that inhibit or stimulate the immune system. In some instances, the immune modulator stimulates CD27+ immune cells. Immune checkpoint pathways and signaling molecules are described in, *e.g.,* Pardoll, Nature Rev Cancer, 2012, 12:252-264; and Mellman et al., Nature, 2011, 480:480- 489.

The immune modulators described herein can be administered at therapeutically effective doses. Therapeutically effective doses can be determined by one of ordinary skill in the art based on the type of immune modulator administered. Dosage, routes of administration, and administration schedules described in the art can be used. Representative doses are available in the Merck Manual Professional Edition (see the internet at merckmanuals.com/professional).

Further, doses of immune modulators administered to animals can be converted to equivalent doses for humans based on the body surface area (BSA) (represented in mg/m2) normalization method (see, e.g., Reagan-Shaw, S. et al., "Dose translation from animal to human studies revisited," FASEB J. 22, 659-661 (2007); and "Guidance for Industry - Estimating the Maximum Safe Starting Dose in Initial Clinical Trials for Therapeutics in Adult Healthy Volunteers," U.S. Department of Health and Human Services, Food and Drug Administration, Center for Drug Evaluation and Research (CDER), July 2005, Pharmacology and Toxicology). For example, the human equivalent dose (HED) based on BSA is can be calculated by the following formula I:

$$\text{I.} \quad \text{HED} = \text{animal dose in mg/kg} \times (\text{animal weight in kg/human weight in kg})0.33$$

Alternatively, the HED can be determined by the following formula II:

$$\text{II.} \quad \text{HED (mg/kg)} = \text{animal dose (mg/kg)} \times (\text{animal Km/human Km})$$

The Km factor is determined based on the following Table (see Guidance for Industry, Id.):

Table 1: Conversion of Animal Doses to Human Equivalent Doses Based on Body Surface Area

| Species | To Convert Animal Dose in mg/kg to Dose in mg/m2, Multiply by $k_m$ | To Convert Animal Dose in mg/kg to HED[a] in mg/kg, Either: | |
| --- | --- | --- | --- |
| | | Divide Animal Dose By | Multiply Animal Dose By |
| Human | 37 | --- | --- |
| Child (20 kg)[b] | 25 | --- | --- |
| Mouse | 3 | 12.3 | 0.08 |
| Hamster | 5 | 7.4 | 0.13 |
| Rat | 6 | 6.2 | 0.16 |
| Ferret | 7 | 5.3 | 0.19 |
| Guinea pig | 8 | 4.6 | 0.22 |
| Rabbit | 12 | 3.1 | 0.32 |
| Dog | 20 | 1.8 | 0.54 |
| Primates: | 12 | 3.1 | 0.32 |
| Monkeys[c] | 6 | 6.2 | 0.16 |
| Marmoset | 7 | 5.3 | 0.19 |
| Squirrel monkey | 20 | 1.8 | 0.54 |
| Baboon | 27 | 1.4 | 0.73 |
| Micro-pig Mini-pig | 35 | 1.1 | 0.95 |

Assumes 60 kg human.

Thus, a 5 mg/kg dose in mice is equivalent to a 0.4 mg/kg dose in a 60 kg human. A 0.4 mg/ml dose in a 60 kg human is equivalent to a dose of 14.8 mg/m2.

In some embodiments, the immune modulators described herein are administered in therapeutically effective amounts for periods of time effective to treat a cancer or tumor. The effective amount of the immune modulators described herein can be determined by one of ordinary skill in the art and includes dosage amounts for a mammal of from about 0.5 to about 200 mg/kg, about 0.5 to about 150 mg/kg, about 0.5 to 100 mg/kg, about 0.5 to about 75mg/kg, about 0.5 to about 50mg/kg, about 0.01 to about 50mg/kg, about 0.05 to about 25 mg/kg, about 0.1 to about 25 mg/kg, about 0.5 to about 25 mg/kg, about 1 to about 20 mg/kg, about 1 to about 10 mg/kg, about 20mg/kg of body weight, about 10 mg/kg, about 5 mg/kg, about 2.5 mg/kg, about 1.0 mg/kg, or about 0.5 mg/kg of body weight of the immune modulator, or any range derivable therein. In some instances of the disclosure, the dosage amounts of the immune modulators are from about 0.01 mg/kg to about 10 mg/kg of body weight. In some instances of the disclosure, the dosage amount of the immune modulator is from about 0.01 mg/kg to about 5 mg/kg, or from about 0.01 mg/kg to about 2.5 mg/kg of body weight. The compositions described herein can be administered in a single dose or in the form of individual divided doses, such as from 1 to 4 times per day, or once every 2 days, 3 days, 4 days, 5 days, 6 days, weekly, or monthly. The compositions described herein can also be administered for various treatment cycles, such as 2, 3, 4, 5, 6, 7, 8, 9, 10 treatment cycles. The treatment cycles can be different lengths of time depending on the cancer to be treated, for example, 1, 2, 3, 4, 5, 6, 7, 8, 9, or 10 week treatment cycles. In addition, the effective amount of an immune modulator described herein can be determined during pre-clinical trials and clinical trials by methods known to physicians and clinicians.

III. Examples

**Example 1: Identifying and using biomarkers to predict response to radiotherapy.**

A radiosensitivity index based on the expression level of one or more molecular biomarkers can be used to predict a cancer patient's sensitivity to radiation. Genomic biomarkers and other indicators of the tumor microenvironment can also be used to predict a patient's response to radiotherapy. Additionally, molecular-target based biomarkers such as CD44 and TGFβ may be predictive of tumor response.

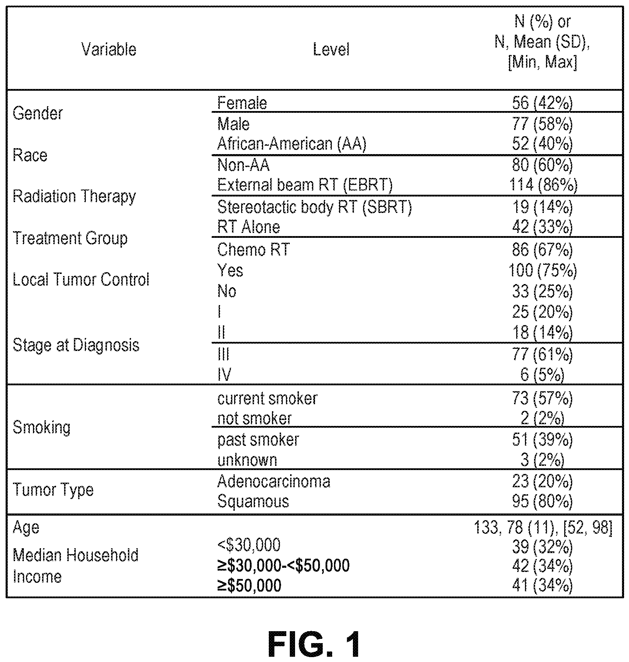

It has been shown that CD44 levels can predict local tumor recurrence after radiotherapy in patients with non-small cell lung cancer (NSCLC). In the study, 133 patients were treated with stereotactic body radiation therapy (SBRT) (12 Gy x 4) or conventional fractionated radiation (60 to 70 Gy) (FIG. 1) (see Kumar, S., et al., "Prognostic Biomarkers in Non-Small Cell Lung Cancer Patients Treated With Radiation Therapy: Locally Advanced Non-Small Cell Lung Cancer," International Journal of Radiation Oncology∗Biology∗Physics, Volume 90, Issue 5, Supplement, 15 November 2014, Pages S25-S26). Tumor samples were obtained from the patient and stained for specific biomarkers including CD44, MFG-E8, and CD68. Analysis of the biomarker expression revealed that CD44 can be used as a biomarker to predict response to radiotherapy.

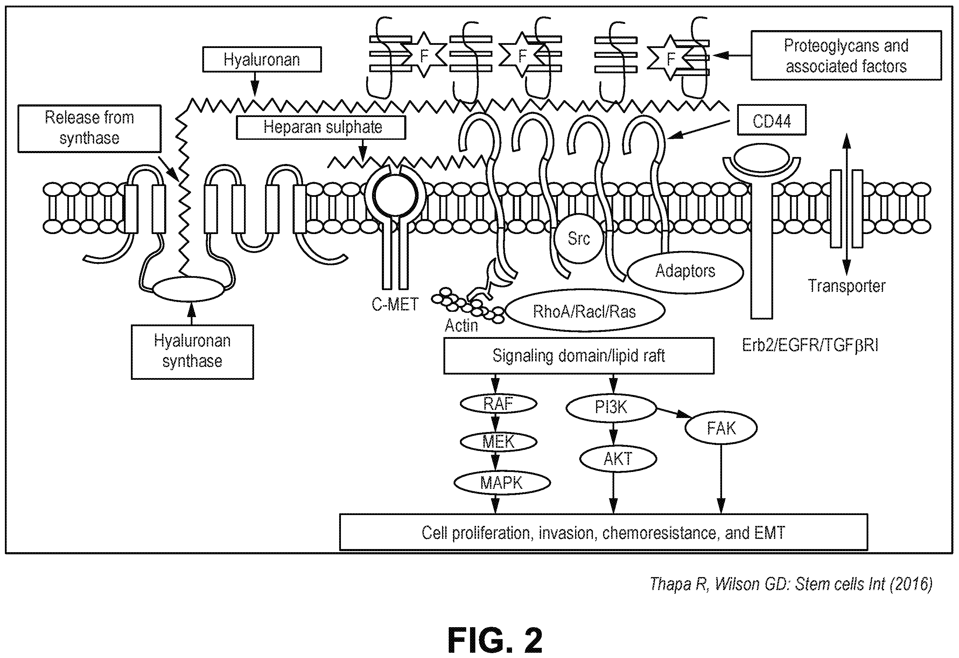

CD44 is a receptor for hyaluronan and is associated with aggressive tumor phenotypes (FIG. 2) (see Thapa R, Wilson GD: Stem cells Int (2016)). It is expressed on cancer initiating cells (CICs) and is involved in TGFβ activation. CD44 has been associated with radioresistance.

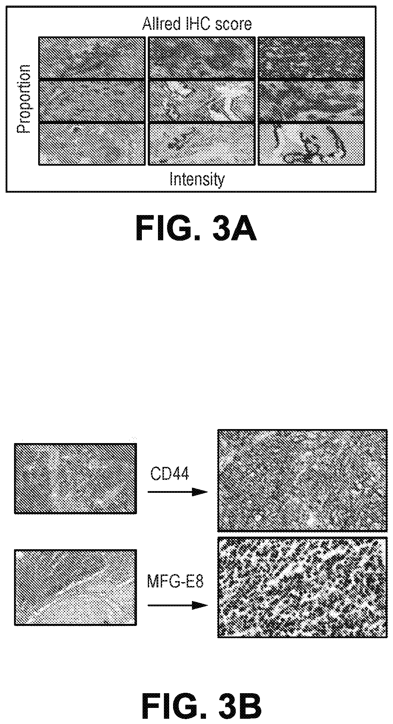

In this study, CD44 protein levesl were quantified according to the Allred scoring system featuring a proportion score and an intensity score to give a total score between 0 and 8 (FIG. 3A). Fig. 3B shows IHC staining of tumor samples with CD44 and MFG-E8. High expression of CD44 and low expression of MFG-E8 were predictive of local tumor control failure. High expression of CD68 was associated with decreased survival benefit to radiotherapy.

Data suggests that the tumor microenvironment may play a role in tumor response to radiotherapy. As such, biomarkers of this microenvironment may be predictive of clinical response.

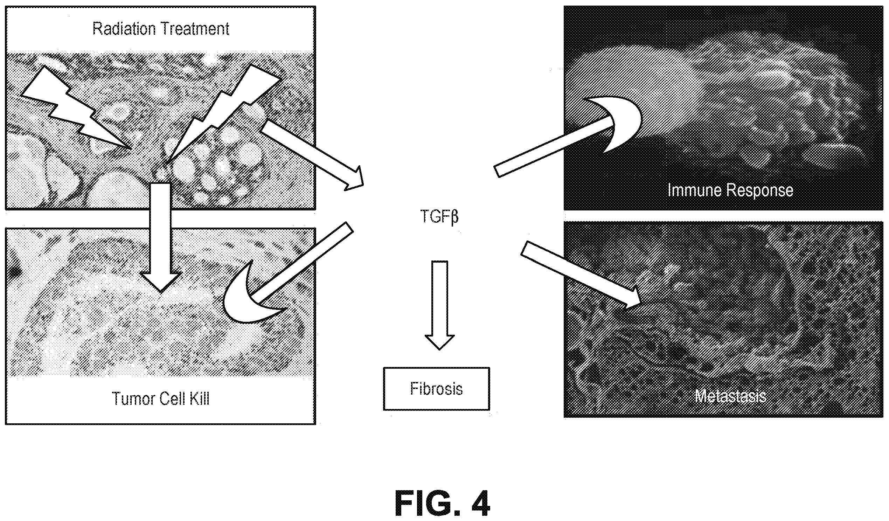

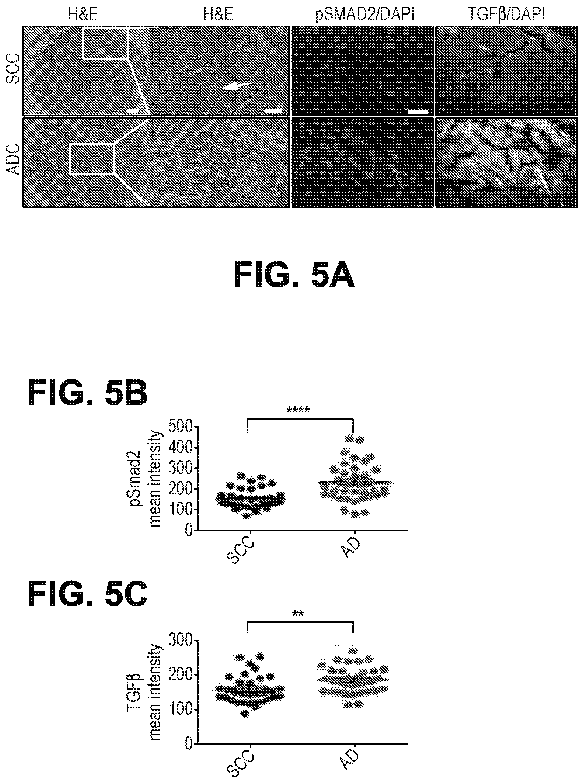

TGFβ is a pleitropic cytokine that is important in normal tissue homeostasis, regulates inflammation and immune responses, and controls proliferation and differentiation. As shown in FIG. 4, there is substantial evidence that TGFβ plays a key role in the response to ionizing radiation. TGFβ is activated in irradiated tissues and plays a role in development of radiation induced fibrosis. It has been shown that TGFβ activity in NSCLC subtypes correlates with a clinical response to radiation. FIG. 5A provides representative images of adenocarcinoma (AD) and small cell lung carcinoma (SCC) human tumors stained with TGFβ and phospho-SMAD2 (a downstream signaling molecule of TGFβ). FIGS. 5B and 5C show that adenocarcinoma tumors express TGFβ at higher levels than SCC tumors. (See Du S, Quyang H, Pellicciotta I, Beheshti A, Lo CH, Parry R, and Barcellos-Hoff MH (2016)).

Biomarkers such as genomic biomarkers, immune cell markers, tumor cell markers, circulating markers, stem cell markers, and the like can be useful for predicting tumor response or sensitivity to radiotherapy. As such, these biomarkers may be expressed in radiation non-responsive or responsive cells and may be indicators of a clinical response to radiotherapy or a lack thereof.

**Example 2: Combination treatment comprising an immune modulator and radiation can enhance inhibition of tumor growth compared to monotherapy.**

In this study female B57/BL6 mice (n=5) were transplanted with MC38 colon carcinoma tumor pieces (2x2mm). The tumors were exposed to gamma radiation (2 Gy) at day 8 post-transplantation. The mice were also admininistered an immune modulator, such as an anti-TIM4 antibody, an anti-MFG-E8 antibody, and an anti-M199 antibody at day 9 and day 11 post-transplantation. 2 mg/kg antibody was injected into each mouse. Tumor volumes were measured along three orthogonal axes (x, y, z) and tumor volume was calculated.

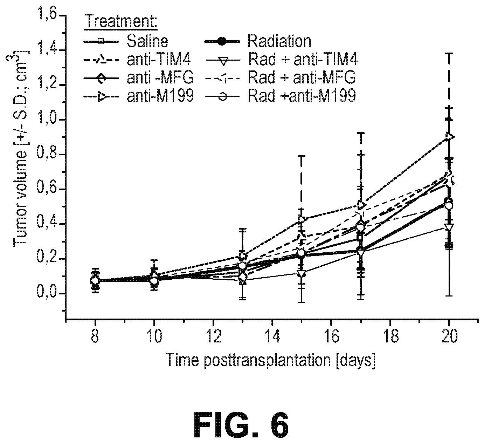

FIG. 6 shows that combination therapy of radiation and an anti-TIM4 antibody resulted in lower tumor growth compared to radiation therapy alone or anti-TIM4 antibody alone. In addition, combination therapy of radiation and an anti-M 199 antibody also shows decreased tumor growth compared to anti-M199 antibody monotherapy. Similarly, anti-MFG-E8 antibody therapy in combination with radiation enhanced tumor growth inhibition compared to monotherapy. The results shows tumor growth inhibition in an immune competent animal model of cancer that has been administered a combination therapy.

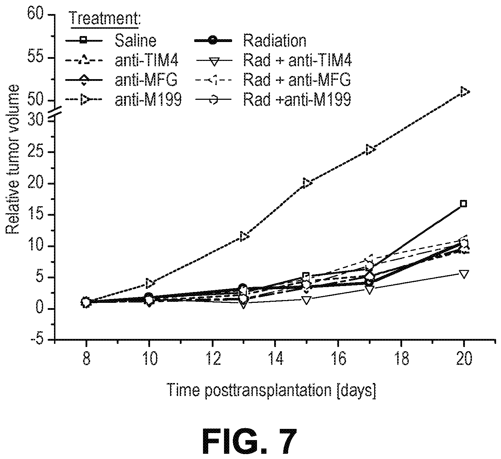

When the relative tumor volume was evaluated, combination therapy comprising radiation and either an anti-TIM4 antibody, anti-MFG-E8 antibody, or an anti-M199 antibody showed enhanced inhibition of tumor growth compared to treatment with an immune modulator alone (FIG. 7). The data shows that radiation in combination with immune modulator therapy can increase the anti-tumor response relative to radiation therapy alone.

**Example 3: TIM-4 and MFGE-8 protein expression levels in human tumor samples**

Material and methods: Formalin-fixed, paraffin-embedded tissue sections were de-paraffinized prior to staining with antibodies targeting either TIM-4 or MFGE-8. The staining was performed using two antigen retrieval methods: TIM-4 - Target Retrieval Solution (Dako), Citrate buffer pH 6.1 at 97°C for 20 minutes; MFGE-8 - Target Retrieval Solution (Dako), Tris EDTA pH 9.0 at 97°C for 20 minutes. Tissue sections were stained using a Dako Envision Flex Kit. Briefly, endogenous peroxides were blocked for 10 minutes with a peroxidase-blocking reagent. For mouse tumor tissues, slides were incubated with peroxidase blocking buffer for 1 hour. Mouse tumor tissue slides were rinsed in washing buffer and then incubated with Fc receptor blocker for 30 minutes. Mouse tissue sections were also incubated using mouse detective (Biocare) for 30 minutes. Tissue sections were incubated with the primary antibody targeting either TIM-4 or MFGE-8 for 30 minutes at RT for human tissues and overnight at 4°C for mouse tissues. Mouse monoclonal antibody MFG-E8 (1/500 for human; Santa Cruz) and Rabbit polyclonal TIM-4 (1/500 for human, 1/400 for mouse; Abcam). Isotype controls and negative controls were run in parallel with respective primary antibodies to rule out any nonspecific staining. Tissues were incubated with the appropriate mouse, rabbit or mouse linker for 10 minutes, washed and then incubated in Dako EnvisionTM + Dual Link System horse radish peroxidase (mouse and rabbit) for 30 minutes. Tissue section were stained for 10 minutes using a DAB chromogen mix and later counterstained with hematoxylin to visualize the nuclei.

Quantification of IHC Expression: The expression of each protein marker was assessed by its intensity and proportion following the methods given below: Briefly, intensity (abbreviated "Int") is scored from 0 to 3 with 0=negative, 1=weak, 2=intermediate, and 3=strong. Proportion (abbreviated "Prop") is scored from 0 to 4 with 0 through 5 corresponding to 0, 1-10, 21-50, 51-80, 81-100 %, respectively. Total score (abbreviated "Tot") is a multiplication of intensity and proportion and has values of 0 -12.

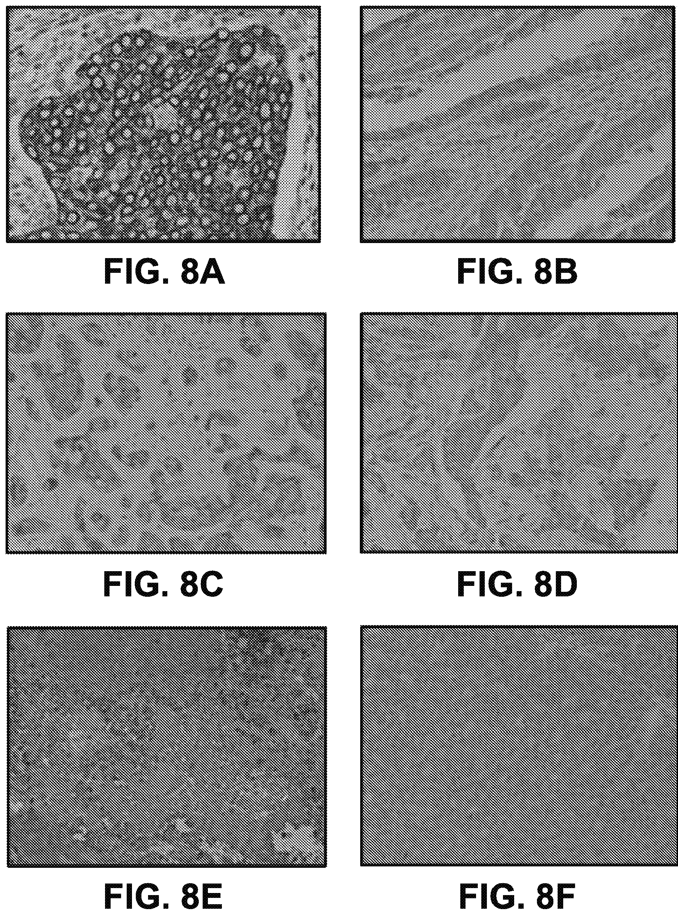

Results: TIM-4 expression was detected in human lung tumors, colon tumors, prostate tumors and breast tumors, and in a tumor bearing syngeneic mouse models, including MC-38 tumor bearing C57/BL6 model (FIG. 8A-8E).

Expression levels of TIM-4 were also evaluated in tumor tissue microarrays (BC041114, LUC481, Biomax, Inc.). TIM-4 expression was evaluated in 106 human lung tumor cases in total. Out of 90 lung tumors (BC041114), 10 cases showed strong staining, 50 cases showed moderate staining, and 30 cases weak staining. Out of 16 human lung tumor cases (LUC481), 4 lung tumor cases showed moderate staining, and 12 cases showed weak staining. TIM-4 expression was also evaluated in a human multi-organ tumor microarray (TMA2001, Biomax Inc.).

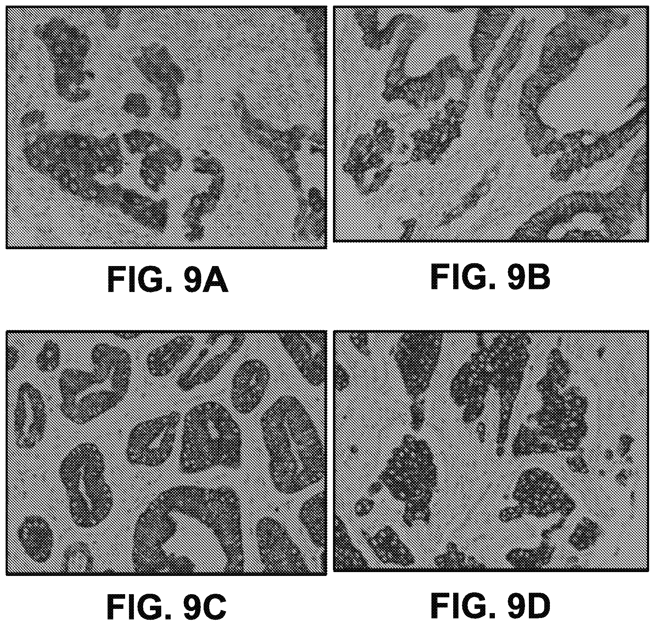

MFGE-8 expression was evaluated in a human multi-organ tumor microarray (TMA 2001, Biomax Inc.) and was detected in multiple tumors, including lung, colon, prostate and breast tumors (FIG. 9A-8D)

**Example 4: Combination treatment comprising an immune modulator and radiation can inhibit tumor growth compared to monotherapy.**

Tumor bearing animals (MC-38 bearing C57/BL6 mice) were treated with either anti-TIM-4 antibody alone (Fig. 10A) or anti-TIM-4 in combination with radiation (Fig. 10B). Fig. 10A shows MC-38 carcinoma bearing mice were treated with anti-TIM4 antibody (2mg/kg) on days 17.19,21,23. Tumor volumes of individual mice (C1-C5) were monitored over the course of the treatment. Fig. 10B shows MC-38 carcinoma bearing mice were treated with radiation (2Gy) at day 16, followed by anti-TIM4 antibody administration (2mg/kg) on days 17.19,21,23. Tumor volumes of individual mice (D1-D5) wer monitored over the course of the treatment. Tumor growth was monitored for up to 50 days. In some cases, as shown in Fig. 10B as an example, the tumor regressed after the initial tumor volume increased.

This example provides additional data showing that treatment of tumors with radiation in combination with an immune modulator can increase the anti-tumor response relative to immune modulator therapy alone.

It is understood that the examples and embodiments described herein are for illustrative purposes only and that various modifications or changes in light thereof will be suggested to persons skilled in the art.

**Claims**

1.  An immune modulator for use in a method of treating a tumor in a subject with cancer comprising administering an effective amount of ionizing radiation and the immune modulator to the tumor, wherein the immune modulator is a small molecule drug, or an antibody or a fragment thereof, that specifically binds to an inhibitory checkpoint molecule and inhibits the activity of the inhibitory checkpoint molecule and the inhibitory checkpoint molecule is TIM4.

2.  An immune modulator for use in a method of treating a tumor in a subject with cancer, according to claim 1, wherein the method further comprises:

    (a) detecting an expression level of one or more biomarkers in a tumor sample from the subject, wherein the one or more biomarkers are selected from the group consisting of an immune cell marker(s), tumor cell marker(s), circulating marker(s), and any combination thereof;
    (b) comparing the expression level of the one or more biomarkers to the expression level of the one or more biomarkers in a normal tissue sample; and
    (c) treating the tumor with ionizing radiation and the immune modulator if the expression level of the one or more biomarkers is modified compared to the expression level in the normal tissue sample.

3.  An immune modulator for use in a method of treating a tumor in a subject with cancer, according to claim 2, wherein the expression level of the one or more biomarkers is modified if the expression level of at least one of the biomarkers is increased, or the expression level of at least one of the biomarkers is decreased, or the expression level of at least one of the biomarkers is increased and the expression level of at least one of the biomarkers is decreased compared to the expression level in a normal tissue sample.

4.  An immune modulator for use in a method of treating a tumor in a subject with cancer, according to claim 2 or Claim 3, wherein the tumor sample is a biopsy comprising tumor cells.

5.  An immune modulator for use in a method of treating a tumor in a subject with cancer, according to any one of claims 2 to 4, wherein the immune cell biomarker(s) or the tumor cell biomarker(s) or the circulating biomarker(s) is a polynucleotide or a protein.

6.  An immune modulator for use in a method of treating a tumor in a subject with cancer, according to claim any one of claims 2 to 5, wherein the biomarker is CD44, MFG-E8, CD68, TGFβ, or a TGFβ-pathway related biomarker.

7.  An immune modulator for use in a method of treating a tumor in a subject with cancer, according to any one of claims 2 to 5, wherein the detecting is performed by using an assay selected from the group consisting of immunohistochemistry, ELISA, Western analysis, HPLC, proteomics, PCR, RT-PCR, Northern analysis, and a microarray.

8.  An immune modulator for use in a method of treating a tumor in a subject with cancer, according to any one of claims 2 to 7, wherein the normal tissue sample comprises non-tumor cells from the same tissue type as the tumor.

9.  An immune modulator for use in a method of treating a tumor in a subject with cancer, according to any one of claims 2 to 8, wherein the expression level of the one or more biomarkers is ranked or weighted.

10. An immune modulator for use in a method of treating a tumor in a subject with cancer, according to any one of claims 2 to 9, further comprising performing functional imaging of the tumor prior to administering the ionizing radiation and the immune modulator.

11. An immune modulator for use in a method of treating a tumor in a subject with cancer, according to any one of claims 2 to 10, wherein the ionizing radiation and/or the immune modulator is administered at a higher dose compared to a standard treatment protocol if the expression level of the one or more biomarkers in the tumor sample is modified compared to the expression level in the normal tissue sample.

12. An immune modulator for use in a method of treating a tumor in a subject with cancer, according to claim 11, wherein the expression level of CD44 is increased and the expression level of MFG-E8 is decreased compared to the expression level in the normal tissue sample.

13. An immune modulator for use in a method of treating a tumor in a subject with cancer, according to claim 11, wherein the expression level of CD68 is increased compared to the expression level in the normal tissue sample.

14. An immune modulator for use in a method of treating a tumor in a subject with cancer, according to any one of claims 2 to 11, wherein the ionizing radiation is administered as a hypofractionated radiation treatment if the expression level of the one or more biomarkers in the tumor sample is modified compared to the expression level in the normal tissue sample.

15. An immune modulator for use in a method of treating a tumor in a subject with cancer, according to any one of claims 2 to 11, wherein the ionizing radiation is administered as a hyperfractionated radiation treatment if the expression level of the one or more biomarkers in the tumor sample is modified compared to the expression level in the normal tissue sample.

**Patentansprüche**

1. Immunmodulator zur Verwendung bei einem Verfahren zum Behandeln eines Tumors bei einem Individuum mit Krebs, das das Verabreichen einer wirksamen Menge ionisierender Strahlung und des Immunodulators an den Tumor umfasst, wobei der Immunmodulator ein kleinmolekulares Arzneimittel oder ein Antikörper oder ein Fragment davon ist, das/der spezifisch an ein hemmendes Checkpoint-Molekül bindet und die Aktivität des hemmenden Checkpoint-Moleküls hemmt, und wobei das hemmende Checkpoint-Molekül TIM4 ist.

2. Immunmodulator zur Verwendung bei einem Verfahren zum Behandeln eines Tumors bei einem Individuum mit Krebs nach Anspruch 1, wobei das Verfahren ferner Folgendes umfasst:

   (a) Detektieren eines Expressionsniveaus eines oder mehrerer Biomarker in einer Tumorprobe von dem Individuum, wobei der eine oder die mehreren Biomarker aus der Gruppe ausgewählt sind, die aus einem Immunzellmarker(n), Tumorzellmarker(n), zirkulierendem Marker/zirkulierenden Markern und einer Kombination daraus besteht;
   (b) Vergleichen des Expressionsniveaus des einen oder der mehreren Biomarker mit dem Expressionsniveau des einen oder der mehreren Biomarker in einer normalen Gewebeprobe; und
   (c) Behandeln des Tumors mit ionisierender Strahlung und dem Immunmodulator, wenn das Expressionsniveau des einen oder der mehreren Biomarker im Vergleich zum Expressionsniveau in der normalen Gewebeprobe modifiziert ist.

3. Immunmodulator zur Verwendung bei einem Verfahren zum Behandeln eines Tumors bei einem Individuum mit Krebs nach Anspruch 2, wobei das Expressionsniveau des einen oder der mehreren Biomarker modifiziert ist, wenn, im Vergleich zum Expressionsniveau in einer normalen Gewebeprobe, das Expressionsniveau mindestens eines der Biomarker erhöht ist oder das Expressionsniveau mindestens eines der Biomarker verringert ist, oder das Expressionsniveau mindestens eines der Biomarker erhöht ist und das Expressionsniveau mindestens eines der Biomarker verringert ist.

4. Immunmodulator zur Verwendung bei einem Verfahren zum Behandeln eines Tumors bei einem Individuum mit Krebs nach Anspruch 2 oder Anspruch 3, wobei die Tumorprobe eine Biopsie, die Tumorzellen umfasst, ist.

5. Immunmodulator zur Verwendung bei einem Verfahren zum Behandeln eines Tumors bei einem Individuum mit Krebs nach einem der Ansprüche 2 bis 4, wobei der/die Immunzell-Biomarker oder der/die Tumorzell-Biomarker oder der/die zirkulierende(n) Biomarker ein Polynucleotid oder ein Protein ist/sind.

6. Immunmodulator zur Verwendung bei einem Verfahren zum Behandeln eines Tumors bei einem Individuum mit Krebs nach einem der Ansprüche 2 bis 5, wobei der Biomarker CD44, MFG-E8, CD68, TGFβ oder ein TGFß-Signalweg-bezogener Biomarker ist.

7. Immunmodulator zur Verwendung bei einem Verfahren zum Behandeln eines Tumors bei einem Individuum mit Krebs nach einem der Ansprüche 2 bis 5, wobei das Detektieren unter Verwendung eines Assays durchgeführt wird, der aus der Gruppe ausgewählt ist, die aus Immunohistochemie-, ELISA-, Western-Analyse, HPLC-, Proteomik-, PCR-, RT-PCR-, Northern-Analyse und einem Microarray besteht.

8. Immunmodulator zur Verwendung bei einem Verfahren zum Behandeln eines Tumors bei einem Individuum mit Krebs nach einem der Ansprüche 2 bis 7, wobei die normale Gewebeprobe Nicht-Tumorzellen von derselben Gewebeart wie der Tumor umfasst.

9. Immunmodulator zur Verwendung bei einem Verfahren zum Behandeln eines Tumors bei einem Individuum mit Krebs nach einem der Ansprüche 2 bis 8, wobei das Expressionsniveau des einen oder der mehreren Biomarker der Rangfolge nach geordnet oder gewichtet ist.

10. Immunmodulator zur Verwendung bei einem Verfahren zum Behandeln eines Tumors bei einem Individuum mit Krebs nach einem der Ansprüche 2 bis 9, ferner das Durchführen einer funktionellen Bildgebung des Tumors vor dem Verabreichen der ionisierenden Strahlung und des Immunmodulators umfassend.

11. Immunmodulator zur Verwendung bei einem Verfahren zum Behandeln eines Tumors bei einem Individuum mit Krebs nach einem der Ansprüche 2 bis 10, wobei die ionisierende Strahlung und/oder der Immunmodulator mit einer höheren Dosis im Vergleich zu einem Standardbehandlungsprotokoll verabreicht wird, wenn das Expressionsniveau des einen oder der mehreren Biomarker in der Tumorprobe im Vergleich zum Expressionsniveau in der normalen Gewebeprobe modifiziert ist.

12. Immunmodulator zur Verwendung bei einem Verfahren zum Behandeln eines Tumors bei einem Individuum mit Krebs nach Anspruch 11, wobei, im Vergleich zum Expressionsniveau in der normalen Gewebeprobe, das Expressionsniveau von CD44 erhöht ist und das Expressionsniveau von MFG-E8 verringert ist.

13. Immunmodulator zur Verwendung bei einem Verfahren zum Behandeln eines Tumors bei einem Individuum mit Krebs nach Anspruch 11, wobei das Expressionsniveau von CD68 im Vergleich zum Expressionsniveau in der normalen Gewebeprobe erhöht ist.

14. Immunmodulator zur Verwendung bei einem Verfahren zum Behandeln eines Tumors bei einem Individuum mit Krebs nach einem der Ansprüche 2 bis 11, wobei die ionisierende Strahlung als eine hypofraktionierte Strahlenbehandlung verabreicht wird, wenn das Expressionsniveau des einen oder der mehreren Biomarker in der Tumorprobe im Vergleich zum Expressionsniveau in der normalen Gewebeprobe modifiziert ist.

15. Immunmodulator zur Verwendung bei einem Verfahren zum Behandeln eines Tumors bei einem Individuum mit Krebs nach einem der Ansprüche 2 bis 11, wobei die ionisierende Strahlung als eine hyperfraktionierte Strahlenbehandlung verabreicht wird, wenn das Expressionsniveau des einen oder der mehreren Biomarker in der Tumorprobe im Vergleich zum Expressionsniveau in der normalen Gewebeprobe modifiziert ist.

**Revendications**

1. Immunomodulateur pour une utilisation dans un procédé de traitement d'une tumeur chez un sujet atteint de cancer comprenant l'administration d'une quantité efficace de rayonnement ionisant et de l'immunomodulateur à la tumeur, dans lequel l'immunomodulateur est un médicament à petite molécule, ou un anticorps ou un fragment de celui-ci, qui se lie spécifiquement à une molécule de point de contrôle inhibiteur et inhibe l'activité de la molécule de point de contrôle inhibiteur et la molécule de point de contrôle inhibiteur est TIM4.

2. Immunomodulateur pour une utilisation dans un procédé de traitement d'une tumeur chez un sujet atteint de cancer, selon la revendication 1, dans lequel le procédé comprend en outre :

   (a) la détection d'un niveau d'expression d'un ou de plusieurs biomarqueurs dans un échantillon de tumeur du sujet, dans lequel les un ou plusieurs biomarqueurs sont choisis dans le groupe constitué d'un ou de marqueurs de cellules immunitaires, d'un ou de marqueurs de cellules tumorales, d'un ou de marqueurs circulants, et de toute combinaison de ceux-ci ;
   (b) la comparaison du niveau d'expression des un ou plusieurs biomarqueurs au niveau d'expression des un ou plusieurs biomarqueurs dans un échantillon de tissu normal ; et
   (c) le traitement de la tumeur avec un rayonnement ionisant et l'immunomodulateur si le niveau d'expression des un ou plusieurs biomarqueurs est modifié par rapport au niveau d'expression dans l'échantillon de tissu normal.

3. Immunomodulateur pour une utilisation dans un procédé de traitement d'une tumeur chez un sujet atteint de cancer, selon la revendication 2, dans lequel le niveau d'expression des un ou plusieurs biomarqueurs est modifié si le niveau d'expression d'au moins un des biomarqueurs est augmenté, ou le niveau d'expression d'au moins un des biomarqueurs est diminué, ou le niveau d'expression d'au moins un des biomarqueurs est augmenté et le niveau d'expression d'au moins un des biomarqueurs est diminué par rapport au niveau d'expression dans un échantillon de tissu normal.

4. Immunomodulateur pour une utilisation dans un procédé de traitement d'une tumeur chez un sujet atteint de cancer, selon la revendication 2 ou la revendication 3, dans lequel l'échantillon de tumeur est une biopsie comprenant des cellules tumorales.

5. Immunomodulateur pour une utilisation dans un procédé de traitement d'une tumeur chez un sujet atteint de cancer, selon l'une quelconque des revendications 2 à 4, dans lequel le ou les biomarqueurs de cellules immunitaires ou le ou les biomarqueurs de cellules tumorales ou le ou les biomarqueurs circulants est/sont un polynucléotide ou une protéine.

6. Immunomodulateur pour une utilisation dans un procédé de traitement d'une tumeur chez un sujet atteint de cancer, selon l'une quelconque des revendications 2 à 5, dans lequel le biomarqueur est CD44, MFG-E8, CD68, TGFβ, ou un biomarqueur lié à la voie TGFβ.

7. Immunomodulateur pour une utilisation dans un procédé de traitement d'une tumeur chez un sujet atteint de cancer, selon l'une quelconque des revendications 2 à 5, dans lequel la détection est effectuée en utilisant un test choisi dans le groupe constitué de l'immunohistochimie, ELISA, l'analyse Western, HPLC, la protéomique, PCR, RT-PCR, l'analyse Northern et un microréseau.

8. Immunomodulateur pour une utilisation dans un procédé de traitement d'une tumeur chez un sujet atteint de cancer, selon l'une quelconque des revendications 2 à 7, dans lequel l'échantillon de tissu normal comprend des cellules non tumorales du même type de tissu que la tumeur.

9. Immunomodulateur pour une utilisation dans un procédé de traitement d'une tumeur chez un sujet atteint de cancer, selon l'une quelconque des revendications 2 à 8, dans lequel le niveau d'expression des un ou plusieurs biomarqueurs est classé ou pondéré.

10. Immunomodulateur pour une utilisation dans un procédé de traitement d'une tumeur chez un sujet atteint de cancer, selon l'une quelconque des revendications 2 à 9, comprenant en outre la réalisation d'une imagerie fonctionnelle de la tumeur avant l'administration du rayonnement ionisant et de l'immunomodulateur.

11. Immunomodulateur pour une utilisation dans un procédé de traitement d'une tumeur chez un sujet atteint de cancer, selon l'une quelconque des revendications 2 à 10, dans lequel le rayonnement ionisant et/ou l'immunomodulateur est/sont administré(s) à une dose plus élevée par rapport à un protocole de traitement standard si le niveau d'expression des un ou plusieurs biomarqueurs dans l'échantillon de tumeur est modifié par rapport au niveau d'expression dans l'échantillon de tissu normal.

12. Immunomodulateur pour une utilisation dans un procédé de traitement d'une tumeur chez un sujet atteint de cancer, selon la revendication 11, dans lequel le niveau d'expression de CD44 est augmenté et le niveau d'expression de MFG-E8 est diminué par rapport au niveau d'expression dans le tissu normal.

13. Immunomodulateur pour une utilisation dans un procédé de traitement d'une tumeur chez un sujet atteint de cancer, selon la revendication 11, dans lequel le niveau d'expression de CD68 est augmenté par rapport au niveau d'expression dans l'échantillon de tissu normal.

14. Immunomodulateur pour une utilisation dans un procédé de traitement d'une tumeur chez un sujet atteint de cancer, selon l'une quelconque des revendications 2 à 11, dans lequel le rayonnement ionisant est administré sous la forme d'un traitement par rayonnement hypofractionné si le niveau d'expression des un ou plusieurs biomarqueurs dans l'échantillon de tumeur est modifié par rapport au niveau d'expression dans l'échantillon de tissu normal.

15. Immunomodulateur pour une utilisation dans un procédé de traitement d'une tumeur chez un sujet atteint de cancer, selon l'une quelconque des revendications 2 à 11, dans lequel le rayonnement ionisant est administré sous la forme d'un traitement par rayonnement hyperfractionné si le niveau d'expression des un ou plusieurs biomarqueurs dans l'échantillon de tumeur est modifié par rapport au niveau d'expression dans l'échantillon de tissu normal.

| Variable | Level | N (%) or N, Mean (SD), [Min, Max] |
|---|---|---|
| Gender | Female | 56 (42%) |
| | Male | 77 (58%) |
| Race | African-American (AA) | 52 (40%) |
| | Non-AA | 80 (60%) |
| Radiation Therapy | External beam RT (EBRT) | 114 (86%) |
| | Stereotactic body RT (SBRT) | 19 (14%) |
| Treatment Group | RT Alone | 42 (33%) |
| | Chemo RT | 86 (67%) |
| Local Tumor Control | Yes | 100 (75%) |
| | No | 33 (25%) |
| Stage at Diagnosis | I | 25 (20%) |
| | II | 18 (14%) |
| | III | 77 (61%) |
| | IV | 6 (5%) |
| Smoking | current smoker | 73 (57%) |
| | not smoker | 2 (2%) |
| | past smoker | 51 (39%) |
| | unknown | 3 (2%) |
| Tumor Type | Adenocarcinoma | 23 (20%) |
| | Squamous | 95 (80%) |
| Age | | 133, 78 (11), [52, 98] |
| Median Household Income | <$30,000 | 39 (32%) |
| | ≥$30,000-<$50,000 | 42 (34%) |
| | ≥$50,000 | 41 (34%) |

# FIG. 1

*Thapa R, Wilson GD: Stem cells Int (2016)*

**FIG. 2**

FIG. 3A

FIG. 3B

FIG. 4

**FIG. 5A**

**FIG. 5B**

**FIG. 5C**

**FIG. 6**

**FIG. 7**

**FIG. 8A**

**FIG. 8B**

**FIG. 8C**

**FIG. 8D**

**FIG. 8E**

**FIG. 8F**

**FIG. 9A**

**FIG. 9B**

**FIG. 9C**

**FIG. 9D**

FIG. 10A

# FIG. 10B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20160024594 A

- US 7801270 B

**Non-patent literature cited in the description**

- **ALLRED, D. C.** *Connection,* 2005, vol. 9, 4-5
- **PARDOLL.** *Nature Rev Cancer,* 2012, vol. 12, 252-264
- **MELLMAN et al.** *Nature,* 2011, vol. 480, 480-489
- **REAGAN-SHAW, S. et al.** Dose translation from animal to human studies revisited. *FASEB J,* 2007, vol. 22, 659-661

- Guidance for Industry - Estimating the Maximum Safe Starting Dose in Initial Clinical Trials for Therapeutics in Adult Healthy Volunteers. *U.S. Department of Health and Human Services, Food and Drug Administration, Center for Drug Evaluation and Research (CDER),* July 2005
- **KUMAR, S. et al.** Prognostic Biomarkers in Non-Small Cell Lung Cancer Patients Treated With Radiation Therapy: Locally Advanced Non-Small Cell Lung Cancer. *International Journal of Radiation Oncology∗Biology∗Physics,* 15 November 2014, vol. 90 (5), S25-S26